# EUROPEAN PATENT SPECIFICATION

(11) **EP 4 497 104 B1**
(45) Date of publication and mention of the grant of the patent: **18.11.2026**
(21) Application number: 23717379.4
(22) Date of filing: 21.03.2023
(51) Int. Cl.: G06V 10/82, G06T 5/60, G06T 5/70, G06V 10/30

(54) **UNCERTAINTY ASSESSMENT OF MEDICAL IMAGE NOISE REDUCTION AND IMAGE PROCESSING**
UNSICHERHEITSBEURTEILUNG VON RAUSCHUNTERDRÜCKUNG UND BILDVERARBEITUNG MEDIZINISCHER BILDER
ÉVALUATION D'INCERTITUDE DE RÉDUCTION DE BRUIT D'IMAGE MÉDICALE ET TRAITEMENT D'IMAGE

(30) Priority: 21.03.2022 US 202263321876 P
(43) Date of publication of application: 29.01.2025
(73) Proprietor: Mayo Foundation for Medical Education and Research, Rochester, Minnesota 55905 (US)
(72) Inventor: HUBER, Nathan R., Rochester, Minnesota 55901 (US); GONG, Hao, Rochester, Wisconsin 55901 (US); YU, Lifeng, Byron, Minnesota 55901 (US); LENG, Shuai, Rochester, Minnesota 55906 (US); MCCOLLOUGH, Cynthia H., Byron, Minnesota 55920 (US)
(74) Representative: Samson & Partner Patentanwälte mbB
(86) International application number: PCT/US2023/064785
(87) International publication number: WO 2023/183821

(56) References cited:
- HUBER NATHAN R ET AL: "Pixel-wise bias approximation and correction for convolutional neural network noise reduction in CT", PROGRESS IN BIOMEDICAL OPTICS AND IMAGING, SPIE - INTERNATIONAL SOCIETY FOR OPTICAL ENGINEERING, BELLINGHAM, WA, US, vol. 12031, 4 April 2022 (2022-04-04), pages 120311C - 120311C, XP060155821, ISSN: 1605-7422, ISBN: 978-1-5106-0027-0, DOI: 10.1117/12.2612703
- WANG GUOTAI ET AL: "Aleatoric uncertainty estimation with test-time augmentation for medical image segmentation with convolutional neural networks", 31 January 2019 (2019-01-31), XP055921220, Retrieved from the Internet <URL:https://arxiv.org/abs/1807.07356> [retrieved on 20220514], DOI: 10.1016/j.neucom.2019.01.103
- WU DUFAN ET AL: "Uncertainty Prediction for Deep Learning-based Image Denoising in Low-dose CT Imaging", 2021 IEEE NUCLEAR SCIENCE SYMPOSIUM AND MEDICAL IMAGING CONFERENCE (NSS/MIC), IEEE, 16 October 2021 (2021-10-16), pages 1 - 2, XP034184213, DOI: 10.1109/NSS/MIC44867.2021.9875474
- YUSUKE NOMURA ET AL: "Calibrated uncertainty estimation for interpretable proton computed tomography image correction using Bayesian deep learning", PHYSICS IN MEDICINE AND BIOLOGY, INSTITUTE OF PHYSICS PUBLISHING, BRISTOL GB, vol. 66, no. 6, 16 March 2021 (2021-03-16), pages 65029, XP020364868, ISSN: 0031-9155, [retrieved on 20210316], DOI: 10.1088/1361-6560/ABE956

## Description

### CROSS-REFERENCE TO RELATED APPLICATIONS

This application claims the benefit of U.S. Provisional Patent Application Serial No. 63/321,876, filed on March 21, 2022, and entitled "Uncertainty Assessment of Medical Image Noise Reduction and Image Processing."

### BACKGROUND

Many artificial intelligence ("AI")-based noise reduction algorithms have been developed during recent years. Popular examples include deep learning reconstruction and image-based convolutional neural network ("CNN") denoising. These highly non-linear algorithms are difficult to assess using standard image quality metrics. For instance, behavior quantified within phantoms generally does not directly correspond to performance in patients. The inability to meaningfully evaluate image quality within patient exams leads to common questions about whether the AI algorithms can be trusted, whether the lesions or other findings in the medical images are real or artifactual creations of the AI algorithms, whether the AI algorithms missed and lesions or other findings in the medical images, and so on.

In general, AI processed images contain errors that could potentially impact reader interpretation. In an effort for accurate diagnostics and transparency, it is desirable that AI-based medical image processing techniques be treated with appropriate consideration of image uncertainty.

Prior literature establishes the importance of quantifying uncertainty within traditionally "black-box" AI algorithms. Including so-called aleatoric and epistemic uncertainty. Aleatoric uncertainty refers to the uncertainty in model output based on randomness during data-generation and epistemic uncertainty refers to uncertainty caused by a lack of knowledge in the algorithm itself.

### Documents

Wang Guotai ET AL: "Aleatoric uncertainty estimation with test-time augmentation for medical image segmentation with convolutional neural networks", 31 January 2019, XP055921220, DOI: 10.1016/j.neucom.2019.01.103
   and
WU DUFAN ET AL: "Uncertainty Prediction for Deep Learning-based Image Denoising in Low-dose CT Imaging",2021 IEEE NUCLEAR SCIENCE SYMPOSIUM AND MEDICAL IMAGING CONFERENCE (NSS/MIC), IEEE, 16 October 2021, pages 1-2, XP034184213,DOI: 10.1109/NSS/MIC44867.2021.9875474 represent the prior art.

### SUMMARY OF THE DISCLOSURE

The present disclosure addresses the aforementioned drawbacks by providing a method for uncertainty assessed medical image noise reduction. The method includes accessing medical image data (e.g., one or more CT images) with a computer system. A noise reduction algorithm is applied to the medical image data using the computer system, generating output as noise-reduced medical image data. Simulated ensemble data are generated from the medical image data by using the computer system to insert noise to the noise-reduced medical image data. In other embodiments, the simulated ensemble data can be generated by inserting noise in the projection domain. For instance, the medical image data can include projection space data and the noise insertion can proceed in the projection domain. The noise reduction algorithm is then applied to the simulated ensemble data using the computer system, generating output as noise-reduced simulated ensemble data. An uncertainty measurement map is generated from the noise-reduced medical image data and the noise-reduced simulated ensemble data. The uncertainty measurement map quantifies an uncertainty of noise reduction in the noise-reduced medical image data. The noise-reduced medical image data and the uncertainty measurement map are displayed to a user or stored for later use (e.g., additional processing).

It is another aspect of the present disclosure to provide a method for uncertainty assessed medical image noise reduction. The method includes accessing medical image data (e.g., one or more CT images) with a computer system. A machine learning algorithm is also accessed with the computer system. The machine learning algorithm has been trained on training data to estimate an uncertainty measurement from a noise-reduced medical image. In other embodiments, the machine learning algorithm has been trained on training data to estimate both an uncertainty measurement and a noise-reduced medical image from the original medical image data. Thus, in some embodiments a noise reduction algorithm is applied to the medical image data using the computer system, generating output as noise-reduced medical image data. An uncertainty measurement map is then generated using the computer system by applying the noise-reduced medical image data to the machine learning algorithm, generating output as the uncertainty measurement map. In other embodiments, original medical image data are applied to the machine learning algorithm, generating output as both the uncertainty measurement map and noise-reduced medical image data. The noise-reduced medical image data and the uncertainty measurement map are displayed to a user and/or stored for later use (e.g., additional processing).

The foregoing and other aspects and advantages of the present disclosure will appear from the following description. In the description, reference is made to the accompanying drawings that form a part hereof, and in which there is shown by way of illustration one or more embodiments. These embodiments do not necessarily represent the full scope of the invention, however, and reference is therefore made to the claims and herein for interpreting the scope of the invention. The invention is, therefore, carried out according to the appended claims.

### BRIEF DESCRIPTION OF THE DRAWINGS

FIG. 1 is a flowchart setting forth the steps of an example ensemble-based method for quantifying uncertainty (e.g., bias and dispersion) in a medical image processing task, such as noise reduction, where the uncertainty measurement data can be used as training data for a machine learning-based uncertainty measurement algorithm.
FIG. 2 is a flowchart setting forth the steps of an example method for generating a bias map from a processed medical image using a bootstrap approximation framework.
FIG. 3 is a schematic illustration of an example bootstrap approximate framework for estimating bias in a CNN-based noise reduction algorithm.
FIG. 4 is a flowchart setting forth the steps of an example method for generating a dispersion map from a processed medical image using a bootstrap approximation framework.
FIG. 5 is a schematic illustration of an example bootstrap approximate framework for estimating bias and dispersion in a CNN-based noise reduction algorithm.
FIG. 6 is an example schematic illustrating the use of a probability loss function in connection with a machine learning algorithm trained to generate a noise-reduced image and a prediction of uncertainty in the noise reduced image.
FIG. 7 shows examples of clinically indicated whole-body-low-dose CT with retrospective CNN noise reduction uncertainty assessment by bootstrap approximation. Extensive uncertainty was observed at bone structure and airways.
FIG. 8 is a block diagram of an example system for estimating uncertainty in a medical image processing task, such as CT image noise reduction.
FIG. 9 is a block diagram of example components that can implement the system of FIG. 8.

### DETAILED DESCRIPTION

Described here are systems and methods for uncertainty assessment of medical image noise reduction and image processing. For instance, the systems and methods allow for quantifying and visualizing the pixel-wise uncertainty, in the form of dispersion and bias, attributable to a convolutional neural network ("CNN"), other deep learning, machine learning, or other artificial intelligence-based noise reduction or other medical image processing techniques. In general, medical images that have been processed with AI-based noise reduction or other medical image processing techniques should be treated with uncertainty that varies from location to location (e.g., pixel-wise uncertainty). Thus, in some embodiments, the systems and methods described in the present disclosure generate and output an uncertainty map that quantifies or otherwise depicts the spatial distribution of uncertainty in a reconstructed or processed medical image. These uncertainty maps provide a confidence map on the information at different locations, organs, tissues, and/or findings in the medical images, thereby providing clinicians with additional information otherwise not available to them for making decisions about or based on the content of the medical images.

At least two classes of uncertainty can be quantified, measured, or otherwise assessed: aleatoric (i.e., data uncertainty) and epistemic (i.e., model uncertainty). Likewise, at least two descriptors of uncertainty can be quantified, measured, or otherwise estimated: dispersion (e.g., variance of repeat measurements) and bias (e.g., difference of a measured value from the actual value).

Broadly speaking, uncertainty parameters can be used to describe dispersion (variance) and/or bias (difference) of a measured value from the actual value. To calculate dispersion or bias conventionally requires a population of measurements. For example, if the same CT scan was repeated 100 times and a CNN noise reduction algorithm applied to each of these images, the dispersion and bias within the ensemble could be determined by direct calculation. However, because patient CT images are only acquired once, uncertainty cannot be calculated using a conventional ensemble measurement approach. The systems and methods described in the present disclosure provide a technical solution to this problem by utilizing a bootstrap approximation and/or deep learning framework for assessing medical image uncertainty. Bootstrapping generally refers to a class of statistical methods that use a measured dataset and internal resampling to approximate the measurement distribution.

Thus, in some implementations, a bootstrap approximation can be used to estimate image uncertainty. In these instances, repeated noise insertion is applied to an individual patient medical image to generate a simulated ensemble. Second, the processing technique is applied to each image in the simulated ensemble. Third, image bias and variance are directly calculated based on processed simulated ensemble.

Additionally or alternatively, deep learning can be used to estimate image uncertainty. As a non-limiting example, a probability loss function framework can be utilized. In this example framework a Gaussian derived probability loss function enables prediction of pixel-wise error bars when training deep learning-based noise reduction algorithms. During inference, this pixel-wise error map can inform the confidence with which each neural network prediction was made.

There remains a need for pixel-wise uncertainty estimation within computed tomography or other medical imaging noise reduction or image processing algorithms. There are several ways in which the uncertainty assessment provided by the systems and methods described in the present disclosure can enable positive clinical impact.

As one advantage, the systems and methods described in the present disclosure can provide transparency regarding image uncertainty. Without measurement uncertainty, it is very difficult to assess how much confidence can be placed in specific regions of a CT image or other medical image. This can be a problem if a radiologist unknowingly makes a diagnosis based on information at highly uncertain regions of the image. Deep learning image processing and iterative reconstruction can generate images of extremely low noise and with extensive artifact correction; however, these processing steps can induce high levels of uncertainty that generally cannot be ascertained when a radiologist views the processed signal alone. Without transparency regarding image uncertainty, there is a risk of incorrect diagnostic decisions being made based on inaccurately processed images.

As another advantage, the systems and methods described in the present disclosure can provide a framework for evaluating medical image processing algorithms, including iterative-based reconstruction and AI-based medical image processing algorithms. Currently, there is a lack of systematic evaluation of non-linear algorithms, including AI-based algorithms and iterative-based algorithms. This is especially an issue with recent advance of AI algorithms, which are usually considered as a "black-box" to the users. The described technique provides a framework for assessing AI algorithms by providing uncertainty at each location of the image so that readers can assess images with confidence levels adjusted accordingly. AI algorithms providing lower uncertainty, in general, are more preferred than ones providing higher uncertainty, which provides an effective way to evaluate AI algorithms using the systems and methods described in the present disclosure.

As still another advantage, the systems and methods described in the present disclosure can provide a framework for developing new AI-based medical image processing algorithms. The described uncertainty assessment techniques may be used by vendors to improve internal noise reduction, image processing, and/or reconstruction algorithms. For example, image processing methods could be optimized to reduce uncertainty in processed images (e.g., cost function for CNN denoising or iterative reconstruction). As another example, noise reduction strength can be tuned based on uncertainty assessment. As yet another example, deep learning noise reduction bias assessment could be used to inform a bias correction.

The systems and methods described in the present disclosure can also be used for optimizing medical imaging protocols. For instance, uncertainty assessment may be helpful when comparing multiple noise reduction and image processing techniques. It is advantageous to implement techniques that have low levels of uncertainty. Likewise, the systems and methods described in the present disclosure can be used for optimizing radiation dose. Radiation dose and medical image uncertainty are inherently linked within x-ray imaging, computed tomography, and other medical imaging modalities that utilize ionizing radiation; dose reduction leads to increased image uncertainty. Uncertainty assessment within medical images may be used to determine the amount of radiation necessary to obtain target levels of confidence within the processed medical image. Target levels of confidence could be established for each diagnostic task.

Described now are systems and methods for quantifying, estimating, or otherwise assessing the uncertainty in a medical image processing task, such as an AI-based image processing task, an iterative reconstruction, or the like.

Within the field of CT imaging, deep learning and other AI-based noise reduction algorithms have demonstrated great potential to improve image quality. As described above, the inability to quantify noise reduction uncertainty could lead to diagnostic errors or nonoptimal image processing.. Thus, there remains a need to be able to quantify or otherwise assess the uncertainty in medical images that are processed using AI-based noise reduction or other image processing algorithms.

In some aspects of the present disclosure, the uncertainty of these methods is characterized using an ensemble measurement framework that is based in part on repeated cadaveric and/or phantom measurements. In these example implementations, multiple measurements of the same cadaveric or phantom region are made, which allows for uncertainty (e.g., bias and dispersion) calculations of the ensemble. These measurements can be used to build models (e.g., statistical models), train algorithms (e.g., machine learning algorithms), or the like.

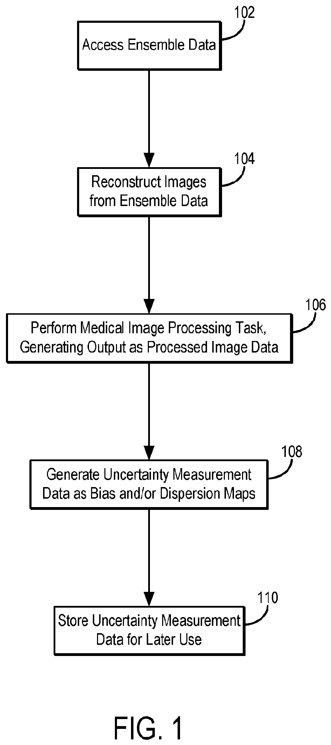

Referring now to FIG. 1, a flowchart is illustrated as setting forth an example method for generating uncertainty maps (e.g., bias maps, dispersion maps) from ensemble data in accordance with some embodiments described in the present disclosure.

The method includes acquiring or otherwise accessing previously acquired ensemble data, as indicated at step 102. As a non-limiting example, the same region of a cadaver or a phantom is imaged multiple times by the medical imaging system, such as a CT imaging system. In one implementation, dynamic sequential scan mode data were acquired with a CT imaging system over one hundred repetitions and no movement of the scanner bed. Repeated images are reconstructed from the ensemble data, as indicated at step 104.

The medical image processing task whose uncertainty is to be assessed is then applied to the images, as indicated at step 106. For example, the medical image processing task can be an AI-based medical image processing task, such as an AI-based noise reduction task. The AI-based noise reduction task can be, for example, based on a machine learning algorithm, such as a CNN or other artificial neural network. In some instances, the medical image processing task whose uncertainty is to be assessed may be an iterative reconstruction algorithm. In these instances, the iterative reconstruction algorithm is applied to the ensemble data in step 106. The resulting images can then be compared with those reconstructed in step 104 (e.g., using a conventional reconstruction algorithm).

The pixel-wise ensemble uncertainty measurements can be calculated from the processed image data, as indicated at step 108. As a non-limiting example, dispersion and bias maps can be generated based on the following equations: $Dispersion Map = \sqrt{\frac{{\sum }_{i = 1}^{n} {\left(CNN\left[{x}_{i}\right]-\overline{CNN \left[x\right]}\right)}^{2}}{n - 1}} ,$ $Bias Map = \frac{{\sum }_{i = 1}^{n} \left({x}_{i}-CNN\left[{x}_{i}\right]\right)}{n} ,$
where *xᵢ* is the *ith* repeated image (i.e., reconstructed image), CNN is the deep learning noise reduction operation (which may be replaced by another suitable AI-based image processing or reconstruction operations), and *n* is the total number of image repetitions (e.g., n = 100). In other embodiments, the uncertainty maps can include confidence maps, precision maps, accuracy maps, and/or variance maps. When the imaging task whose uncertainty to be assessed is an image reconstruction, then the image, *xᵢ*, can be reconstructed using a conventional reconstruction method and the image, *CNN*[*xᵢ*], can be the image reconstructed using the reconstruction technique whose uncertainty is being assessed.

These uncertainty measurements (i.e., dispersion maps and bias maps) can be stored for later use, as indicated at step 110. For instance, the dispersion and bias maps can be stored and used as training data for a machine learning algorithm that is trained to estimate dispersion and bias in a single medical image, to construct a statistical model, or so on.

Similarly, the dispersion and bias maps can also be used to assess the tested image processing technique (e.g., a CNN noise reduction algorithm). For instance, using the ensemble of cadaver and/or phantom images, the pixel-wise dispersion and bias of the CNN noise reduction algorithm can be computed. The dispersion and bias maps can be analyzed to assess the image processing task. For example, in an example study, elevated dispersion was observed within regions of high image gradient; commonly associated with cortical bone, calcification, and air pockets. In the same study, elevated bias was observed primarily in regions containing fine and high contrast image features, such as trabecular bone and lung airways. This assessment indicated extensive degradation in CNN denoised signal compared to a one-hundred repetition ensemble average reference. Structure loss was most apparent for fine details within trabecular bone and soft tissue. Ensemble measurement uncertainty was calculated for the cadaveric exams. For uniform tissue, dispersion was on the order of 15 HU and bias was negligible. For high contrast structures, dispersion and absolute bias routinely exceeded 50 HU.

As another example, during diagnostic medical image review, the radiologist can be presented processed medical image data along with the corresponding uncertainty map(s) (e.g., dispersion map, bias map, confidence map, precision map, accuracy map, or variance map). The processed medical image data and uncertainty map(s) can be used by the radiologist to make a diagnosis and inform associated diagnostic confidence. Additionally or alternatively, the radiologist can also be presented with automated annotations or indicators of low-confidence regions, as determined or otherwise informed by the uncertainty map(s).

In other example, the uncertainty map(s) (e.g., confidence map, precision map, accuracy map, or variance map) can be incorporated into a loss function, cost function, and/or objective function used in a medical image processing algorithm during training (or model optimization).

Upon close inspection of CNN denoised images, loss of contrast at fine or low contrast structures is often observed. Systematic bias within CNN noise reduction algorithms could lead to misinformed diagnostic decisions if not appropriately quantified. Thus, it is one aspect of the present disclosure to provide a bootstrap estimation method that can be used to approximate the pixel-wise bias inflicted by CNN noise reduction on patient CT images, or other AI-based image processing algorithms on other medical images. In general, the bootstrap approximation framework includes a simulated ensemble and bootstrap approximation to estimate the bias within CT noise reduction on an image-specific basis. Prior noise reduction and image processing algorithms are unable to approximate bias associated with CNN noise reduction.

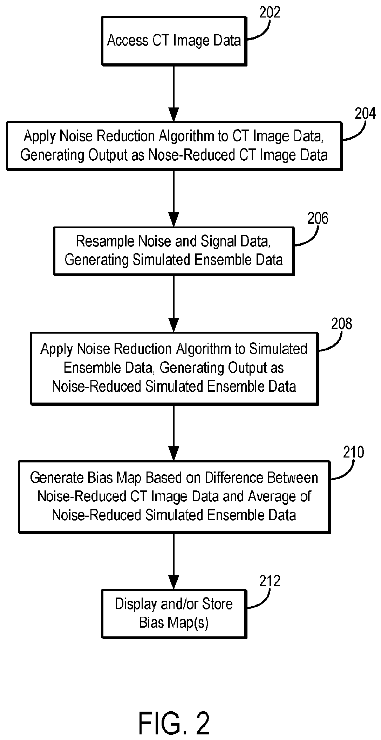

As a non-limiting example, the bias approximation framework can make use of bootstrap methods in statistics. Referring now to FIG. 2, a flowchart is illustrated as setting forth the steps of an example method for generating a bias map in accordance with some embodiments described in the present disclosure. In general, the bias approximation is applied on an image-specific basis.

Medical image data are accessed with a computer system, as indicated at step 202. The medical image data may be data and/or images acquired with a medical imaging system, such as a CT imaging system. The medical image data can be accessed by retrieving previously acquired medical image data from a memory or other data storage device or medium. Additionally or alternatively, accessing the medical image data can include acquiring the medical image data with the medical imaging system and communicating the acquired medical image data to the computer system, which in some instances may be a part of the medical imaging system.

A medical image processing algorithm is then applied to the medical image data using the computer system, as indicated at step 204. For example, the medical image processing algorithm may be a noise reduction algorithm. The noise reduction algorithm may be an AI-based noise reduction algorithm, such as a CNN-based noise reduction algorithm. Additionally or alternatively, the noise reduction algorithm can be implemented as part of an iterative reconstruction algorithm. The output of the noise reduction algorithm includes an approximation of a noise-free signal (S') and noise-only image (N') from the CT scan of interest.

The noise and signal data are then resampled by applying a local spatial decoupling operation (Ω) to the noise-only image, in which the noise is randomly translated and inverted prior to being added back to the noise-free signal, S', as indicated at step 206. This resampling operation can be repeatedly applied to obtain multiple unique representations of the CT image of interest. The unique images generated in this manner can be referred to as a simulated ensemble. Additionally or alternatively, the simulated ensemble data can be generated using repeatedly applied projection noise insertion (i.e., noise insertion in the projection domain) to obtain multiple unique noise representations of the CT image of interest.

The medical image processing task (e.g., the CNN noise reduction algorithm) is then applied to the simulated ensemble, as indicated at step 208. Bias introduced by the medical image processing task is calculated by taking the difference between the average processed (e.g., CNN denoised) simulated ensemble and the original noise-free image approximation, generating output as one or more bias maps, as indicated at step 210. The bias map(s) can then be displayed to a user or stored for later use (e.g., to correct for the bias in the noise-reduced images), as indicated at step 212. For instance, the bias map(s) can be displayed to a user (e.g., a radiologist) so that the radiologist can review the bias map(s) with the original image in order to decide on a level of confidence in their diagnosis. These maps can be used to not only visualize, but also correct the CT images. This uncertainty quantification can also be used for AI algorithm development (e.g., by helping to determine how to train an algorithm so that output uncertainty is minimized), and radiation dose optimization (e.g., by helping determine a minimum dose compatible with a target level of confidence).

In some instances, residual noise may be present within the bias approximation due to incomplete removal of noise by the CNN when approximating the noise-free signal. A dot product can be used to determine the correlation coefficient between the noise-free approximation (N') and bias map to remove residual noise from the bias map.

In some instances, with data obtained using a bootstrap evaluation, a CNN can be trained to estimate bias directly on the original image without repeating the bootstrap evaluation for each patient. For example, the method described with respect to FIG. 1 can be used to generate a training data set that is then used to train a CNN or other machine learning algorithm to estimate a bias map from a single processed medical image. In these instances, the medical image data accessed with the computer system are applied to the medical image processing task (e.g., a CNN-based noise reduction algorithm) and the output image(s) are applied to the trained machine learning algorithm, generating output as the bias map(s).

In some implementations, image-based noise insertion is used. Additionally or alternatively, projection-based noise insertion with multiple repetitions can also be used. This technique is described for CNN noise reduction, but a similar process could be used to predict uncertainty in iterative reconstruction.

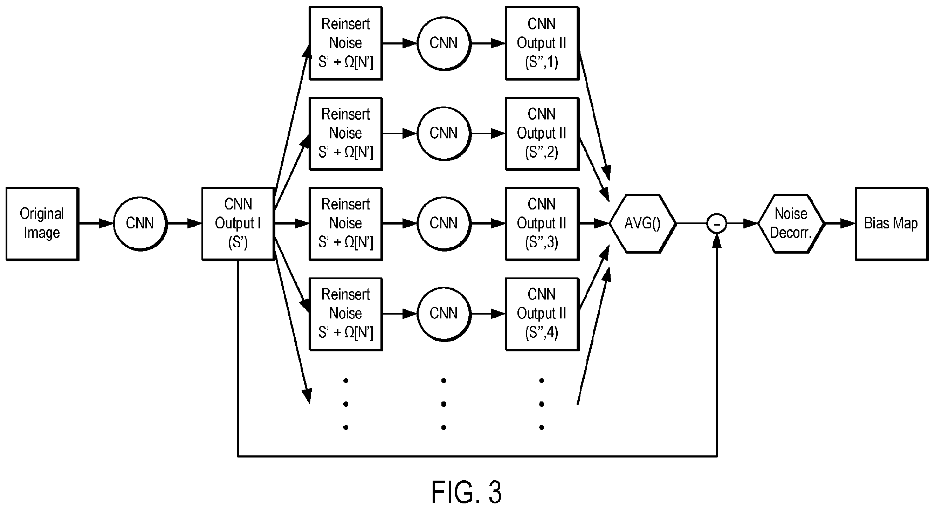

FIG. 3 illustrates an example schematic of a bias approximation technique for a CNN noise reduction algorithm. The CNN is first applied to original image to approximate noise-free signal (S') and noise-only image (N'). A spatial decoupling operator (Ω) is applied to the noise-only image and then added to the noise-free signal to form a simulated ensemble of 100 images. The CNN is applied to each image in the simulated ensemble. The denoised simulated ensemble is assessed in terms of pixel-wise bias relative to initial noise-free signal (S'). Noise decorrelation relative to the noise-only image (N') was used to remove residual noise from the bias map.

In addition to pixel-wise bias approximation, the systems and methods described in the present disclosure can also provide for pixel-wise dispersion approximation, which can be used to determine regions of high and low uncertainty within processed medical images. Advantageously, this dispersion information can be used for algorithm development (e.g., cost function minimizing uncertainty), informing clinical diagnosis (e.g., encouraging diagnostic decisions in regions of low uncertainty), among other uses.

As a non-limiting example, a simulated ensemble and bootstrap approximation can be used to estimate the dispersion within CT noise reduction, or other medical image processing task, on an image-specific basis. Prior deep learning image processing algorithms are unable to approximate dispersion associated with CNN noise reduction.

Bootstrapping is a term used for metrics based on random sampling with replacement. In the dispersion approximation framework, multiple noise insertions on single image can be used to generate a simulated ensemble. The CNN (or other AI-based image processing task) can be applied to the simulated ensemble and the dispersion in response can be calculated.

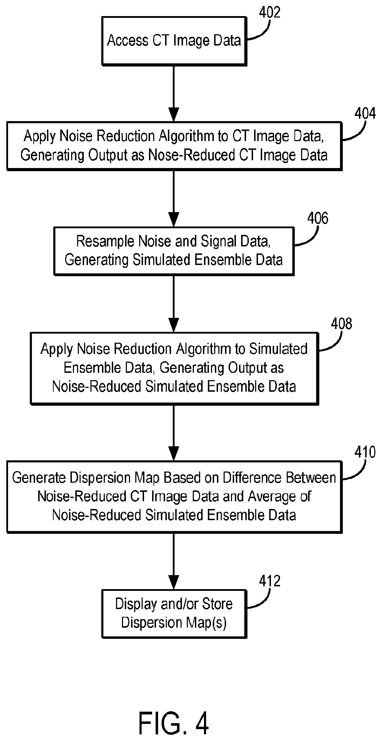

Referring now to FIG. 4, a flowchart is illustrated as setting forth the steps of an example method for generating a dispersion map in accordance with some embodiments described in the present disclosure.

Medical image data are accessed with a computer system, as indicated at step 402. The medical image data may be data and/or images acquired with a medical imaging system, such as a CT imaging system. The medical image data can be accessed by retrieving previously acquired medical image data from a memory or other data storage device or medium. Additionally or alternatively, accessing the medical image data can include acquiring the medical image data with the medical imaging system and communicating the acquired medical image data to the computer system, which in some instances may be a part of the medical imaging system.

A medical image processing algorithm is then applied to the medical image data using the computer system, as indicated at step 404. For example, the medical image processing algorithm may be a noise reduction algorithm. The noise reduction algorithm may be an AI-based noise reduction algorithm, such as a CNN-based noise reduction algorithm. Additionally or alternatively, the noise reduction algorithm can be implemented as part of an iterative reconstruction algorithm. The output of the noise reduction algorithm includes an approximation of a noise-free signal (S') and noise-only image (N') from the CT scan of interest. The noise-only image represents a quantification of the pixel-wise image noise level in CT image, and the noise-free signal represents an approximate signal ground truth.

The noise is then reinserted into the signal ground truth signal (i.e., the CNN denoised image) following the local noise level prediction, generating simulated ensemble data, as indicated at step 406. As a non-limiting example, the simulated ensemble can correspond to 100 noise reinserted images. Alternatively, the noise insertion can be performed in the projection space.

The medical image processing task (e.g., the CNN noise reduction algorithm) is then applied to the simulated ensemble, generating noise-reduced simulated ensemble data, as indicated at step 408. One or more dispersion maps are then generated by computing the pixel-wise dispersion that occurred within the simulated ensemble, as indicated at step 410. The dispersion map(s) can be displayed to a user, or stored for later, as indicated at step 412. For instance, the dispersion map(s) can be displayed to a user (e.g., a radiologist) so that the radiologist can review the dispersion map(s) with the original image in order to decide on a level of confidence in their diagnosis. These maps can be used to not only visualize, but also correct the CT images. This uncertainty quantification can also be used for AI algorithm development (e.g., by helping to determine how to train an algorithm so that output uncertainty is minimized), and radiation dose optimization (e.g., by helping determine a minimum dose compatible with a target level of confidence).

In some instances, with data obtained using a bootstrap evaluation, a CNN can be trained to estimate dispersion directly on the original image without repeating the bootstrap evaluation for each patient. For example, the method described with respect to FIG. 1 can be used to generate a training data set that is then used to train a CNN or other machine learning algorithm to estimate a dispersion map from a single processed medical image. In these instances, the medical image data accessed with the computer system are applied to the medical image processing task (e.g., a CNN-based noise reduction algorithm) and the output image(s) are applied to the trained machine learning algorithm, generating output as the dispersion map(s).

In some implementations, image-based noise insertion is used. Additionally or alternatively, projection-based noise insertion with multiple repetitions can also be used. This technique is described for CNN noise reduction, but a similar process could be used to predict uncertainty in iterative reconstruction.

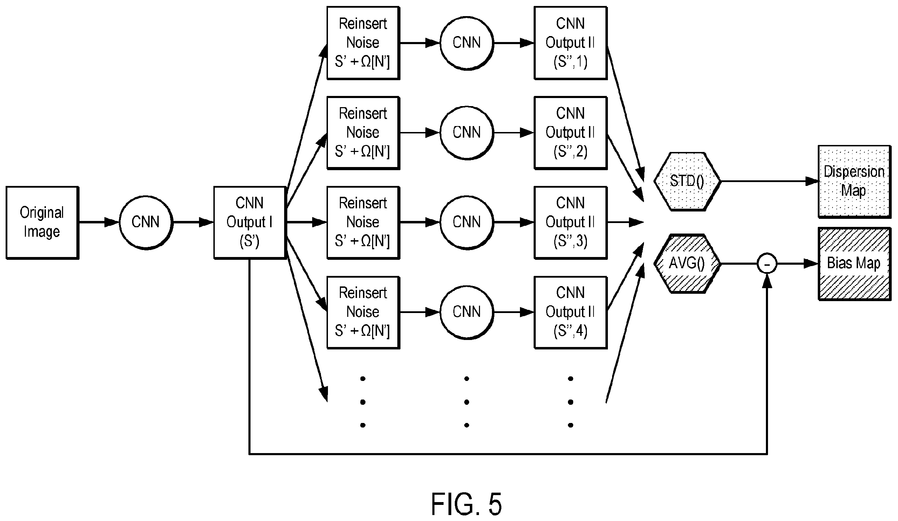

FIG. 5 illustrates a schematic of bias and dispersion approximation technique for a CNN noise reduction algorithm. The CNN is first applied to original image to approximate noise-free signal (S') and noise-only image (N'). A spatial decoupling operator (Ω) is applied to the noise-only image and then added to the noise-free signal to form a simulated ensemble of 100 images. CNN is applied to each image in the simulated ensemble. The denoised simulated ensemble is assessed in terms of pixel-wise bias and dispersion relative to initial noise-free signal (S').

Currently, noise reduction methods (e.g., CNN-based and iterative reconstruction-based) are trained based on loss functions that quantify signal accuracy relative to some ground truth image or data fidelity constraint. In some embodiments, the systems and methods described in the present disclosure overcome these previous drawback by incorporating loss functions that are capable of predicting both target signal and dispersion, which as a non-limiting example may be derived from the Gaussian probability distribution function. This Gaussian-derived probability loss function is complementary to the dispersion approximation framework described above, and may be used additionally or alternatively to that framework. In the dispersion approximation framework described above, the dispersion calculation is independent of the training process. Incorporating dispersion calculation into the loss function can provide an additional benefit for network optimization.

As a non-limiting example, noise in a CT image can be closely approximated by a Gaussian distribution with the following probability density function: $f \left(x, σ\right) = \frac{1}{σ \sqrt{2 π}} {e}^{- \frac{1}{2} {\left(\frac{x - μ}{σ}\right)}^{2}}$
where x is the measurement, *µ* is the expected value, and σ is the standard deviation in the measurement. The probability density function is maximized when x and *µ* are similar and *σ* is low, which can be used as a cost function as follows: $\begin{matrix}x , σ = {argmax}_{x , σ} \left[\frac{1}{σ \sqrt{2 π}}{e}^{- \frac{1}{2} {\left(\frac{x - μ}{σ}\right)}^{2}}\right] \\ = {argmax}_{x , σ} \left[ln\left(\frac{1}{σ \sqrt{2 π}}\right)-\frac{1}{2}{\left(\frac{x - μ}{σ}\right)}^{2}\right] \\ = {argmax}_{x , σ} \left[ln\left(1\right)-ln\left(σ\right)-ln\left(\sqrt{2 π}\right)-\frac{1}{2}{\left(\frac{x - μ}{σ}\right)}^{2}\right] \\ = {argmax}_{x , σ} \left[-ln\left(σ\right)-\frac{1}{2}{\left(\frac{x - μ}{σ}\right)}^{2}\right]\end{matrix}$ $Probability Loss = {argmin}_{x , σ} \left[ln\left(σ\right)+\frac{1}{2}{\left(\frac{x - μ}{σ}\right)}^{2}\right] .$

During training, the expected value (*µ*) is replaced with the training target image. The neural network output image is *x*, and *σ* is an additional output that reflects the dispersion in the predicted value. It can be seen that the probability loss will decrease as the neural network output image (x) approaches the expectation value (*µ*). Additionally, the probability loss will decrease as the dispersion in this prediction (a) decreases. This leads the network to make accurate predictions with high confidence whenever possible.

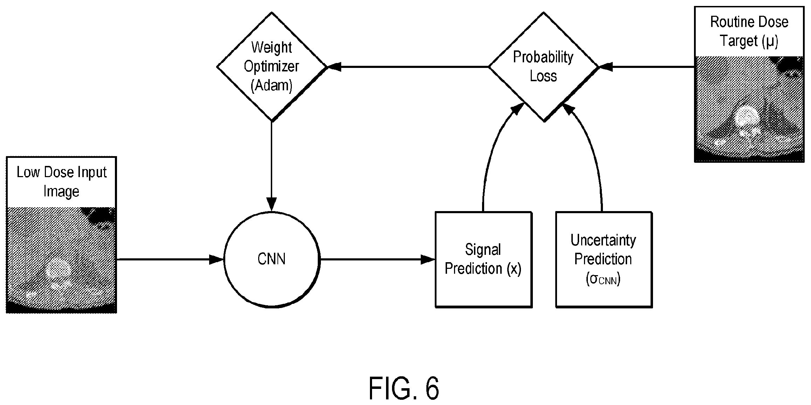

One alteration to neural network architecture that allows for use of this probabilistic loss function is having a two-channel output. These two channels contain predictions for the signal and dispersion. The probability loss can be calculated on a pixel-wise basis in conjunction with the routine dose target. FIG. 6 illustrates a schematic of an example neural network incorporating probability loss. The CNN acts on a low dose CT input to produce two images containing the signal prediction (x) as well as the uncertainty prediction (σ_{CNN}).

In an example study, a CNN noise reduction technique developed for whole-body-low-dose (WBLD) CT skeletal survey was assessed using the systems and methods described in the present disclosure. The architecture used resembled U-Net with a mean-squared-error loss function. Training data for the CNN was generated using an image-based noise insertion procedure designed to be easily implemented on any CT scanner. Noise-only image realizations were obtained from repeated sampling of an anthropomorphic phantom. Throughout the training process, noise-only image patches were randomly superimposed on routine WBLD-CT patient images to synthesize data for training via supervised learning.

The training inputs included noise inserted WBLD-CT patches and training targets were the same images without noise insertion. Following training, the CNN was applied to routine WBLD-CT exams for the purpose of noise reduction and associated uncertainty quantification.

Repeated cadaver scans were acquired for the purpose of uncertainty calculation by ensemble measurement, as described above. Five cadavers were scanned with one-hundred repetitions. A dynamic sequential scan mode was used such that repeated scans were acquired with no movement of the scanner bed and with the same starting tube angle. Cadaver scans were performed with 120 kV following a routine whole-body-low-dose CT protocol (70 effective mAs) and reconstructed with filtered-back-projection using a medium-sharp kernel (Br64).

Thirty clinically indicated WBLD-CT patient exams were obtained for retrospective evaluation of CNN noise reduction uncertainty. Ten of these exams were reserved for CNN training and evaluation, the remaining exams were used for bootstrap uncertainty approximation. Scans were acquired with 120 kV and 70 effective mAs and reconstructed using a medium-sharp kernel (Br64).

Using the repeated cadaver dataset, pixel-wise uncertainty (dispersion and bias) of CNN noise reduction was measured by ensemble measurement. To calculate dispersion, CNN noise reduction was applied to each of the one-hundred cadaver acquisitions. Then, the pixel-wise ensemble standard deviation of the denoised images was calculated; calculation for ensemble dispersion was defined as, $Dispersion Map = \sqrt{\frac{{\sum }_{i = 1}^{n} {\left(CNN\left[{x}_{i}\right]-\overline{CNN \left[x\right]}\right)}^{2}}{n}} ,$
where *xᵢ* is the *i*^{th} repeated cadaver FBP image, *CNN* is the deep learning noise reduction operation, and n is the total number of image repetitions (in this case n = 100). To calculate ensemble measurement bias, CNN noise reduction is similarly applied to each of the one-hundred cadaver acquisitions. Then, the ensemble difference between FBP input and CNN denoised output was calculated; calculation for ensemble bias was defined as, $Bias Map = \frac{{\sum }_{i = 1}^{n} \left({x}_{i}-CNN\left[{x}_{i}\right]\right)}{n} .$

Because patient CT exams are only acquired once, uncertainty cannot be calculated using an ensemble measurement. The bootstrap and/or deep learning methodologies described in the present disclosure can instead be used. For example, the bootstrap methodology utilizes repeated noise insertion of a single CT exam to form a simulated ensemble for the purpose of uncertainty approximation. As another example, a deep learning methodology can implement a machine learning algorithm trained on ensemble or simulated ensemble data, such that a single CT image can be input to the trained machine learning algorithm to output the uncertainty measurement data (e.g., bias and/or dispersion maps). In some instances, the machine learning algorithm can implement both the medical image processing task (e.g., noise reduction) and generate the uncertainty measurement of that image processing task. For example, a CT image can be input to the trained machine learning algorithm, generating output as a noise-reduced CT image in addition to a bias map and/or dispersion map.

To calculate bootstrap uncertainty, the methods described above were implemented. For example, first CNN noise reduction was applied to an individual patient exam to approximate the noise-free signal (S') and noise-only image (N'). The noise-only image was then reinserted into the noise-free signal one-hundred times using a spatial decoupling operation (Ω). Spatial decoupling was defined as a random noise inversion and a random translation of noise relative to signal by a radius of 1 to 15 pixels in any direction. Because the noise and signal were spatially decoupled, the simulated ensemble of one-hundred noise-reinserted images were unique to the original CT image. After noise insertion, CNN noise reduction was applied to the simulated ensemble. Once a simulated ensemble was obtained, dispersion and bias were calculated in the same manner as was done with cadaver ensemble measurement and described above in more detail.

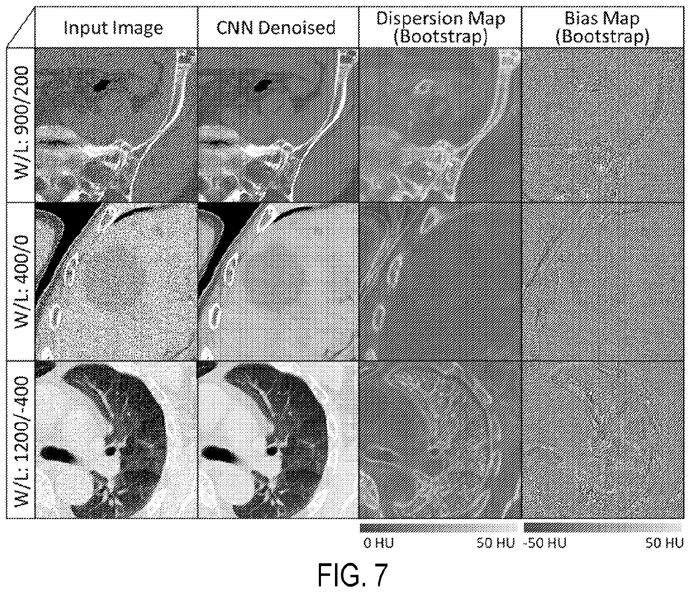

FIG. 7 shows examples of clinically indicated whole-body-low-dose CT with retrospective CNN noise reduction uncertainty assessment by bootstrap approximation. Extensive uncertainty was observed at bone structure and airways.

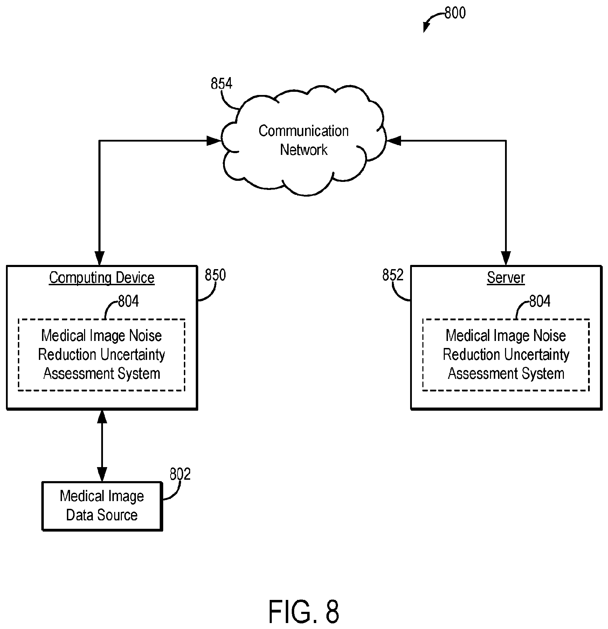

Referring now to FIG. 8, an example of a system 800 for noise reduction uncertainty assessment in accordance with some embodiments of the systems and methods described in the present disclosure is shown. As shown in FIG. 8, a computing device 850 can receive one or more types of data (e.g., medical image data) from medical image data source 802, which may be an CT image data source. In some embodiments, computing device 850 can execute at least a portion of a medical image noise reduction uncertainty measurement system 804 to estimate uncertainty measurements (e.g., bias and/or dispersion) from medical image data (e.g., CT images) that have been processed with a medical image processing algorithm, such as an AI-based noise reduction algorithm, which may be received from the medical image data source 802.

Additionally or alternatively, in some embodiments, the computing device 850 can communicate information about data received from the image source 802 to a server 852 over a communication network 854, which can execute at least a portion of the medical image noise reduction uncertainty measurement system 804. In such embodiments, the server 852 can return information to the computing device 850 (and/or any other suitable computing device) indicative of an output of the medical image noise reduction uncertainty measurement.

In some embodiments, computing device 850 and/or server 852 can be any suitable computing device or combination of devices, such as a desktop computer, a laptop computer, a smartphone, a tablet computer, a wearable computer, a server computer, a virtual machine being executed by a physical computing device, and so on. The computing device 850 and/or server 852 can also reconstruct images from the data.

In some embodiments, medical image data source 802 can be any suitable source of data (e.g., measurement data, medical images reconstructed from measurement data, processed medical image data), such as a medical imaging system (e.g., a CT imaging system), another computing device (e.g., a server storing medical image data), and so on. In some embodiments, medical image data source 802 can be local to computing device 850. For example, medical image data source 802 can be incorporated with computing device 850 (e.g., computing device 850 can be configured as part of a device for measuring, recording, estimating, acquiring, or otherwise collecting or storing data). As another example, medical image data source 802 can be connected to computing device 850 by a cable, a direct wireless link, and so on. Additionally or alternatively, in some embodiments, medical image data source 802 can be located locally and/or remotely from computing device 850, and can communicate data to computing device 850 (and/or server 852) via a communication network (e.g., communication network 854).

In some embodiments, communication network 854 can be any suitable communication network or combination of communication networks. For example, communication network 854 can include a Wi-Fi network (which can include one or more wireless routers, one or more switches, etc.), a peer-to-peer network (e.g., a Bluetooth network), a cellular network (e.g., a 3G network, a 4G network, etc., complying with any suitable standard, such as CDMA, GSM, LTE, LTE Advanced, WiMAX, etc.), other types of wireless network, a wired network, and so on. In some embodiments, communication network 854 can be a local area network, a wide area network, a public network (e.g., the Internet), a private or semi-private network (e.g., a corporate or university intranet), any other suitable type of network, or any suitable combination of networks. Communications links shown in FIG. 8 can each be any suitable communications link or combination of communications links, such as wired links, fiber optic links, Wi-Fi links, Bluetooth links, cellular links, and so on.

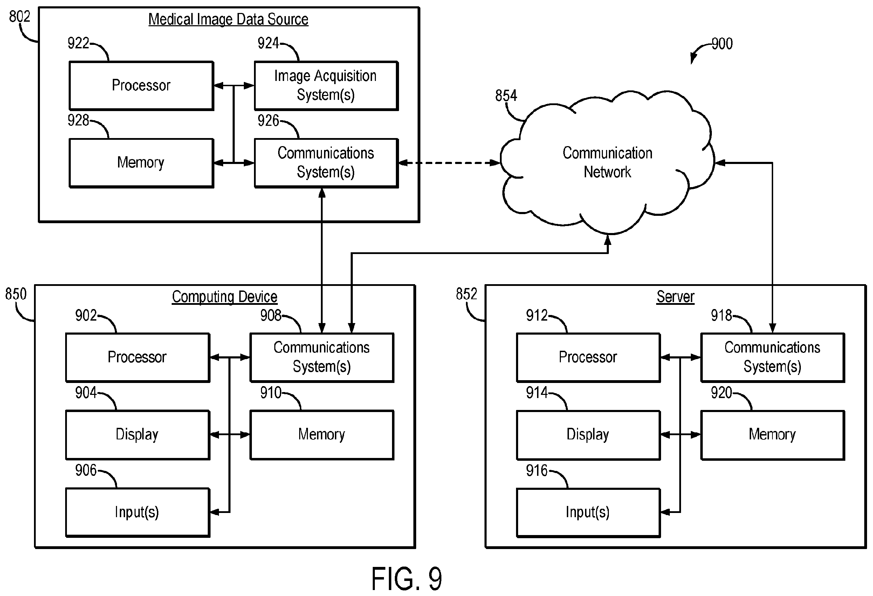

Referring now to FIG. 9, an example of hardware 900 that can be used to implement medical image data source 802, computing device 850, and server 852 in accordance with some embodiments of the systems and methods described in the present disclosure is shown.

As shown in FIG. 9, in some embodiments, computing device 850 can include a processor 902, a display 904, one or more inputs 906, one or more communication systems 908, and/or memory 910. In some embodiments, processor 902 can be any suitable hardware processor or combination of processors, such as a central processing unit ("CPU"), a graphics processing unit ("GPU"), and so on. In some embodiments, display 904 can include any suitable display devices, such as a liquid crystal display ("LCD") screen, a light-emitting diode ("LED") display, an organic LED ("OLED") display, an electrophoretic display (e.g., an "e-ink" display), a computer monitor, a touchscreen, a television, and so on. In some embodiments, inputs 906 can include any suitable input devices and/or sensors that can be used to receive user input, such as a keyboard, a mouse, a touchscreen, a microphone, and so on.

In some embodiments, communications systems 908 can include any suitable hardware, firmware, and/or software for communicating information over communication network 854 and/or any other suitable communication networks. For example, communications systems 908 can include one or more transceivers, one or more communication chips and/or chip sets, and so on. In a more particular example, communications systems 908 can include hardware, firmware, and/or software that can be used to establish a Wi-Fi connection, a Bluetooth connection, a cellular connection, an Ethernet connection, and so on.

In some embodiments, memory 910 can include any suitable storage device or devices that can be used to store instructions, values, data, or the like, that can be used, for example, by processor 902 to present content using display 904, to communicate with server 852 via communications system(s) 908, and so on. Memory 910 can include any suitable volatile memory, non-volatile memory, storage, or any suitable combination thereof. For example, memory 910 can include random-access memory ("RAM"), read-only memory ("ROM"), electrically programmable ROM ("EPROM"), electrically erasable ROM ("EEPROM"), other forms of volatile memory, other forms of non-volatile memory, one or more forms of semi-volatile memory, one or more flash drives, one or more hard disks, one or more solid state drives, one or more optical drives, and so on. In some embodiments, memory 910 can have encoded thereon, or otherwise stored therein, a computer program for controlling operation of computing device 850. In such embodiments, processor 902 can execute at least a portion of the computer program to present content (e.g., images, user interfaces, graphics, tables), receive content from server 852, transmit information to server 852, and so on. For example, the processor 902 and the memory 910 can be configured to perform the methods described herein (e.g., the method of FIG. 1; the method of FIG. 2; the method of FIG. 4).

In some embodiments, server 852 can include a processor 912, a display 914, one or more inputs 916, one or more communications systems 918, and/or memory 920. In some embodiments, processor 912 can be any suitable hardware processor or combination of processors, such as a CPU, a GPU, and so on. In some embodiments, display 914 can include any suitable display devices, such as an LCD screen, LED display, OLED display, electrophoretic display, a computer monitor, a touchscreen, a television, and so on. In some embodiments, inputs 916 can include any suitable input devices and/or sensors that can be used to receive user input, such as a keyboard, a mouse, a touchscreen, a microphone, and so on.

In some embodiments, communications systems 918 can include any suitable hardware, firmware, and/or software for communicating information over communication network 854 and/or any other suitable communication networks. For example, communications systems 918 can include one or more transceivers, one or more communication chips and/or chip sets, and so on. In a more particular example, communications systems 918 can include hardware, firmware, and/or software that can be used to establish a Wi-Fi connection, a Bluetooth connection, a cellular connection, an Ethernet connection, and so on.

In some embodiments, memory 920 can include any suitable storage device or devices that can be used to store instructions, values, data, or the like, that can be used, for example, by processor 912 to present content using display 914, to communicate with one or more computing devices 850, and so on. Memory 920 can include any suitable volatile memory, non-volatile memory, storage, or any suitable combination thereof. For example, memory 920 can include RAM, ROM, EPROM, EEPROM, other types of volatile memory, other types of non-volatile memory, one or more types of semi-volatile memory, one or more flash drives, one or more hard disks, one or more solid state drives, one or more optical drives, and so on. In some embodiments, memory 920 can have encoded thereon a server program for controlling operation of server 852. In such embodiments, processor 912 can execute at least a portion of the server program to transmit information and/or content (e.g., data, images, a user interface) to one or more computing devices 850, receive information and/or content from one or more computing devices 850, receive instructions from one or more devices (e.g., a personal computer, a laptop computer, a tablet computer, a smartphone), and so on.

In some embodiments, the server 852 is configured to perform the methods described in the present disclosure. For example, the processor 912 and memory 920 can be configured to perform the methods described herein (e.g., the method of FIG. 1; the method of FIG. 2; the method of FIG. 4).

In some embodiments, medical image data source 802 can include a processor 922, one or more data acquisition systems 924, one or more communications systems 926, and/or memory 928. In some embodiments, processor 922 can be any suitable hardware processor or combination of processors, such as a CPU, a GPU, and so on. In some embodiments, the one or more data acquisition systems 924 are generally configured to acquire data, images, or both, and can include a medical imaging system, such as a CT imaging system. Additionally or alternatively, in some embodiments, the one or more data acquisition systems 924 can include any suitable hardware, firmware, and/or software for coupling to and/or controlling operations of a medical imaging system. In some embodiments, one or more portions of the data acquisition system(s) 924 can be removable and/or replaceable.

Note that, although not shown, medical image data source 802 can include any suitable inputs and/or outputs. For example, medical image data source 802 can include input devices and/or sensors that can be used to receive user input, such as a keyboard, a mouse, a touchscreen, a microphone, a trackpad, a trackball, and so on. As another example, medical image data source 802 can include any suitable display devices, such as an LCD screen, an LED display, an OLED display, an electrophoretic display, a computer monitor, a touchscreen, a television, etc., one or more speakers, and so on.

In some embodiments, communications systems 926 can include any suitable hardware, firmware, and/or software for communicating information to computing device 850 (and, in some embodiments, over communication network 854 and/or any other suitable communication networks). For example, communications systems 926 can include one or more transceivers, one or more communication chips and/or chip sets, and so on. In a more particular example, communications systems 926 can include hardware, firmware, and/or software that can be used to establish a wired connection using any suitable port and/or communication standard (e.g., VGA, DVI video, USB, RS-232, etc.), Wi-Fi connection, a Bluetooth connection, a cellular connection, an Ethernet connection, and so on.

In some embodiments, memory 928 can include any suitable storage device or devices that can be used to store instructions, values, data, or the like, that can be used, for example, by processor 922 to control the one or more data acquisition systems 924, and/or receive data from the one or more data acquisition systems 924; to generate images from data; present content (e.g., images, a user interface) using a display; communicate with one or more computing devices 850; and so on. Memory 928 can include any suitable volatile memory, non-volatile memory, storage, or any suitable combination thereof. For example, memory 928 can include RAM, ROM, EPROM, EEPROM, other types of volatile memory, other types of non-volatile memory, one or more types of semi-volatile memory, one or more flash drives, one or more hard disks, one or more solid state drives, one or more optical drives, and so on. In some embodiments, memory 928 can have encoded thereon, or otherwise stored therein, a program for controlling operation of medical image data source 802. In such embodiments, processor 922 can execute at least a portion of the program to generate images, transmit information and/or content (e.g., data, images) to one or more computing devices 850, receive information and/or content from one or more computing devices 850, receive instructions from one or more devices (e.g., a personal computer, a laptop computer, a tablet computer, a smartphone, etc.), and so on.

In some embodiments, any suitable computer-readable media can be used for storing instructions for performing the functions and/or processes described herein. For example, in some embodiments, computer-readable media can be transitory or non-transitory. For example, non-transitory computer-readable media can include media such as magnetic media (e.g., hard disks, floppy disks), optical media (e.g., compact discs, digital video discs, Blu-ray discs), semiconductor media (e.g., RAM, flash memory, EPROM, EEPROM), any suitable media that is not fleeting or devoid of any semblance of permanence during transmission, and/or any suitable tangible media. As another example, transitory computer-readable media can include signals on networks, in wires, conductors, optical fibers, circuits, or any suitable media that is fleeting and devoid of any semblance of permanence during transmission, and/or any suitable intangible media.

The present disclosure has described one or more preferred embodiments, and it should be appreciated that many equivalents, alternatives, variations, and modifications, aside from those expressly stated, are possible and within the scope of the appended claims.

## Claims

1. A method for uncertainty assessed medical image noise reduction, the method comprising:
(a) accessing medical image data with a computer system;
(b) applying a noise reduction algorithm to the medical image data using the computer system, generating output as noise-reduced medical image data and noise-only medical image data;
(c) generating simulated ensemble data from the medical image data by using the computer system to repeatedly insert different noise to the noise-reduced medical image data, wherein each different noise is computed by applying a different local spatial decoupling operation to the noise-only image;
(d) applying the noise reduction algorithm to the simulated ensemble data using the computer system, generating output as noise-reduced simulated ensemble data;
(e) generating an uncertainty measurement map from the noise-reduced medical image data and the noise-reduced simulated ensemble data, the uncertainty measurement map quantifying an uncertainty of noise reduction in the noise-reduced medical image data; and
(f) displaying the noise-reduced medical image data and the uncertainty measurement map to a user.

2. The method of claim 1, wherein the uncertainty measurement map comprises a bias map.

3. The method of claim 2, wherein the bias map is computed as a difference between the noise-reduced medical image data and an average of the noise-reduced simulated ensemble data.

4. The method of claim 1, wherein the uncertainty measurement map comprises a dispersion map.

5. The method of claim 4, wherein the dispersion map is computed as a pixel-wise standard deviation amongst the noise-reduced simulated ensemble data.

6. The method of claim 1, wherein the uncertainty measurement map comprises both a bias map and a dispersion map.

7. The method of claim 1, wherein step (f) comprises:
generating corrected noise-reduced medical image data with the computer system by correcting the noise-reduced medical image data for the uncertainty of noise reduction in the noise-reduced medical image data using the uncertainty measurement map; and
displaying the noise-reduced medical image data and the uncertainty measurement map to the user comprises displaying the corrected noise-reduced medical image data to the user.

8. The method of claim 1, wherein the noise reduction algorithm is a convolutional neural network-based noise reduction algorithm.

9. A method for uncertainty assessed medical image noise reduction, the method comprising:
(a) accessing medical image data with a computer system;
(b) accessing a machine learning algorithm with the computer system, wherein the machine learning algorithm has been trained on training data to estimate an uncertainty measurement from a noise-reduced medical image, wherein the training data comprise uncertainty measurement maps generated by:
applying a noise reduction algorithm to a medical image, generating output as a noise-reduced medical image and a noise-only medical image;
generating simulated ensemble data by repeatedly inserting different noise to the noise-reduced medical image, wherein each different noise is computed by applying a different local spatial decoupling operation to the noise-only image;
applying the noise reduction algorithm to the simulated ensemble data, generating output as noise-reduced simulated ensemble data; and
generating the uncertainty measurement maps from the noise-reduced medical image and the noise-reduced simulated ensemble data;
(c) applying the noise reduction algorithm to the medical image data using the computer system, generating output as noise-reduced medical image data;
(d) generating an uncertainty measurement map using the computer system by applying the noise-reduced medical image data to the machine learning algorithm, generating output as the uncertainty measurement map; and
(e) displaying the noise-reduced medical image data and the uncertainty measurement map to a user.

10. The method of any one of claims 1 or 9, wherein the medical image data comprise computed tomography (CT) image data.

11. The method of claim 10, wherein the CT image data consists of a single CT image.

12. The method of claim 9, wherein the uncertainty measurement map comprises at least one of a bias map or a dispersion map.

13. The method of claim 9, wherein the machine learning algorithm is trained using a probability loss.

14. The method of claim 13, wherein the probability loss is a Gaussian derived probability loss function.

15. The method of claim 9, wherein the noise reduction algorithm is a convolutional neural network-based noise reduction algorithm.

## Patentansprüche

1. Verfahren zur Rauschunterdrückung in medizinischen Bildern unter Berücksichtigung von Unsicherheiten, wobei das Verfahren umfasst:
(a) Abrufen medizinischer Bilddaten mit einem Computersystem;
(b) Anwenden eines Rauschunterdrückungsalgorithmus auf die medizinischen Bilddaten unter Verwendung des Computersystems, wodurch als Ausgabe rauschreduzierte medizinische Bilddaten und reine medizinische Rauschbilddaten erzeugt werden;
(c) Erzeugen simulierter Ensemble-Daten aus den medizinischen Bilddaten, indem mithilfe des Computersystems wiederholt unterschiedliches Rauschen in die rauschreduzierten medizinischen Bilddaten eingefügt wird, wobei jedes unterschiedliche Rauschen durch Anwenden einer unterschiedlichen lokalen räumlichen Entkopplungsoperation auf das reine Rauschbild berechnet wird;
(d) Anwenden des Rauschunterdrückungsalgorithmus auf die simulierten Ensemble-Daten mithilfe des Computersystems, wodurch als Ausgabe rauschreduzierte simulierte Ensemble-Daten erzeugt werden;
(e) Erzeugen einer Unsicherheitsmesskarte aus den rauschreduzierten medizinischen Bilddaten und den rauschreduzierten simulierten Ensemble-Daten, wobei die Unsicherheitsmesskarte eine Unsicherheit der Rauschreduktion in den rauschreduzierten medizinischen Bilddaten quantifiziert; und
(f) Anzeigen der rauschreduzierten medizinischen Bilddaten und der Unsicherheitsmesskarte für einen Benutzer.

2. Verfahren nach Anspruch 1, wobei die Unsicherheitsmesskarte eine Bias-Karte umfasst.

3. Verfahren nach Anspruch 2, wobei die Bias-Karte als Differenz zwischen den rauschreduzierten medizinischen Bilddaten und einem Mittelwert der rauschreduzierten simulierten Ensemble-Daten berechnet wird.

4. Verfahren nach Anspruch 1, wobei die Unsicherheitsmesskarte eine Streuungskarte umfasst.

5. Verfahren nach Anspruch 4, wobei die Streuungskarte als pixelweise Standardabweichung unter den rauschreduzierten simulierten Ensemble-Daten berechnet wird.

6. Verfahren nach Anspruch 1, wobei die Unsicherheitsmesskarte sowohl eine Bias-Karte als auch eine Streuungskarte umfasst.

7. Verfahren nach Anspruch 1, wobei Schritt (f) umfasst:
Erzeugen korrigierter, rauschreduzierter medizinischer Bilddaten mit dem Computersystem durch Korrigieren der rauschreduzierten medizinischen Bilddaten hinsichtlich der Unsicherheit der Rauschreduktion in den rauschreduzierten medizinischen Bilddaten unter Verwendung der Unsicherheitsmesskarte; und
das Anzeigen der rauschreduzierten medizinischen Bilddaten und der Unsicherheitsmesskarte für den Benutzer umfasst das Anzeigen der korrigierten rauschreduzierten medizinischen Bilddaten für den Benutzer.

8. Verfahren nach Anspruch 1, wobei der Rauschunterdrückungsalgorithmus ein auf einem Faltungsneuronalen Netzwerk basierender Rauschunterdrückungsalgorithmus ist.

9. Verfahren zur Rauschunterdrückung in medizinischen Bildern unter Berücksichtigung von Unsicherheiten, wobei das Verfahren umfasst:
(a) Abrufen medizinischer Bilddaten mit einem Computersystem;
(b) Zugreifen auf einen Algorithmus für maschinelles Lernen mittels des Computersystems, wobei der Algorithmus für maschinelles Lernen anhand von Trainingsdaten trainiert wurde, um eine Unsicherheitsmessung aus einem rauschreduzierten medizinischen Bild zu schätzen, wobei die Trainingsdaten Unsicherheitsmesskarten umfassen, die erzeugt wurden durch:
Anwenden eines Rauschunterdrückungsalgorithmus auf ein medizinisches Bild, wobei als Ausgabe ein rauschreduziertes medizinisches Bild und ein reines medizinisches Rauschbild erzeugt werden;
Erzeugen simulierter Ensemble-Daten durch wiederholtes Einfügen unterschiedlichen Rauschens in das rauschreduzierte medizinische Bild, wobei jedes unterschiedliche Rauschen durch Anwenden einer unterschiedlichen lokalen räumlichen Entkopplungsoperation auf das reine Rauschbild berechnet wird;
Anwenden des Rauschunterdrückungsalgorithmus auf die simulierten Ensemble-Daten, wodurch als Ausgabe rauschreduzierte simulierte Ensemble-Daten erzeugt werden; und
Erzeugen der Unsicherheitsmesskarten aus dem rauschreduzierten medizinischen Bild und den rauschreduzierten simulierten Ensemble-Daten;
(c) Anwenden des Rauschunterdrückungsalgorithmus auf die medizinischen Bilddaten unter Verwendung des Computersystems, wodurch als Ausgabe rauschreduzierte medizinische Bilddaten erzeugt werden;
(d) Erzeugen einer Unsicherheitsmesskarte mithilfe des Computersystems, indem die rauschreduzierten medizinischen Bilddaten auf den Algorithmus für maschinelles Lernen angewendet werden, wobei als Ausgabe die Unsicherheitsmesskarte erzeugt wird; und
(e) Anzeigen der rauschreduzierten medizinischen Bilddaten und der Unsicherheitsmesskarte für einen Benutzer.

10. Verfahren nach Anspruch 1 oder 9, wobei die medizinischen Bilddaten Computertomographie-Bilddaten (CT-Bilddaten) umfassen.

11. Verfahren nach Anspruch 10, wobei die CT-Bilddaten aus einem einzelnen CT-Bild bestehen.

12. Verfahren nach Anspruch 9, wobei die Unsicherheitsmesskarte eine Bias-Karte und/oder eine Streuungskarte umfasst.

13. Verfahren nach Anspruch 9, wobei der Algorithmus für maschinelles Lernen unter Verwendung eines Wahrscheinlichkeitsverlusts trainiert wird.

14. Verfahren nach Anspruch 13, wobei der Wahrscheinlichkeitsverlust eine von der Gaußschen Verteilung abgeleitete Wahrscheinlichkeitsverlustfunktion ist.

15. Verfahren nach Anspruch 9, wobei der Rauschunterdrückungsalgorithmus ein auf einem Faltungsneuronalen Netzwerk basierender Rauschunterdrückungsalgorithmus ist.

## Revendications

1. Méthode de réduction de bruit d'image médicale à évaluation d'incertitude, la méthode comprenant :
(a) l'accès à des données d'image médicale avec un système informatique ;
(b) l'application d'un algorithme de réduction de bruit aux données d'image médicale à l'aide du système informatique, générant une sortie sous forme de données d'image médicale à bruit réduit et de données d'image médicale à bruit uniquement ;
(c) la génération de données d'ensemble simulées à partir des données d'image médicale à l'aide du système informatique pour insérer de manière répétée un bruit différent dans les données d'image médicale à bruit réduit, dans laquelle chaque bruit différent est calculé par application d'une opération de découplage spatial local différente à l'image à bruit uniquement ;
(d) l'application de l'algorithme de réduction de bruit aux données d'ensemble simulées à l'aide du système informatique, générant une sortie sous forme de données d'ensemble simulées à bruit réduit ;
(e) la génération d'une carte de mesure d'incertitude à partir des données d'image médicale à bruit réduit et des données d'ensemble simulées à bruit réduit, la carte de mesure d'incertitude quantifiant une incertitude de réduction de bruit dans les données d'image médicale à bruit réduit ; et
(f) l'affichage des données d'image médicale à bruit réduit et de la carte de mesure d'incertitude à un utilisateur.

2. Méthode selon la revendication 1, dans laquelle la carte de mesure d'incertitude comprend une carte de biais.

3. Méthode selon la revendication 2, dans laquelle la carte de biais est calculée comme une différence entre les données d'image médicale à bruit réduit et une moyenne des données d'ensemble simulées à bruit réduit.

4. Méthode selon la revendication 1, dans laquelle la carte de mesure d'incertitude comprend une carte de dispersion.

5. Méthode selon la revendication 4, dans laquelle la carte de dispersion est calculée comme un écart-type par pixel parmi les données d'ensemble simulées à bruit réduit.

6. Méthode selon la revendication 1, dans laquelle la carte de mesure d'incertitude comprend à la fois une carte de biais et une carte de dispersion.

7. Méthode selon la revendication 1, dans laquelle l'étape (f) comprend :
la génération de données d'image médicale à bruit réduit corrigées avec le système informatique par correction des données d'image médicale à bruit réduit pour l'incertitude de réduction de bruit dans les données d'image médicale à bruit réduit à l'aide de la carte de mesure d'incertitude ; et
l'affichage des données d'image médicale à bruit réduit et de la carte de mesure d'incertitude à l'utilisateur comprend l'affichage des données d'image médicale à bruit réduit corrigées à l'utilisateur.

8. Méthode selon la revendication 1, dans laquelle l'algorithme de réduction de bruit est un algorithme de réduction de bruit basé sur un réseau neuronal convolutif.

9. Méthode de réduction de bruit d'image médicale à évaluation d'incertitude, la méthode comprenant :
(a) l'accès à des données d'image médicale avec un système informatique ;
(b) l'accès à un algorithme d'apprentissage automatique avec le système informatique, dans laquelle l'algorithme d'apprentissage automatique a été entraîné sur des données d'entraînement pour estimer une mesure d'incertitude à partir d'une image médicale à bruit réduit, dans laquelle les données d'entraînement comprennent des cartes de mesure d'incertitude générées par :
application d'un algorithme de réduction de bruit à une image médicale, générant une sortie sous la forme d'une image médicale à bruit réduit et d'une image médicale à bruit uniquement ;
génération de données d'ensemble simulées par insertion répétée de bruits différents dans l'image médicale à bruit réduit, dans laquelle chaque bruit différent est calculé par application d'une opération de découplage spatial local différente à l'image à bruit uniquement ;
application de l'algorithme de réduction de bruit aux données d'ensemble simulées, générant une sortie sous forme de données d'ensemble simulées à bruit réduit ; et
génération des cartes de mesure d'incertitude à partir de l'image médicale à bruit réduit et des données d'ensemble simulées à bruit réduit ;
(c) l'application de l'algorithme de réduction de bruit aux données d'image médicale à l'aide du système informatique, générant une sortie sous forme de données d'image médicale à bruit réduit ;
(d) la génération d'une carte de mesure d'incertitude à l'aide du système informatique par application des données d'image médicale à bruit réduit à l'algorithme d'apprentissage automatique, générant une sortie sous la forme de la carte de mesure d'incertitude ; et
(e) l'affichage des données d'image médicale à bruit réduit et de la carte de mesure d'incertitude à un utilisateur.

10. Méthode selon l'une quelconque des revendications 1 ou 9, dans laquelle les données d'image médicale comprennent des données d'image de tomodensitométrie (TDM).

11. Méthode selon la revendication 10, dans laquelle les données d'image TDM consistent en une seule image TDM.

12. Méthode selon la revendication 9, dans laquelle la carte de mesure d'incertitude comprend au moins l'une parmi une carte de biais ou une carte de dispersion.

13. Méthode selon la revendication 9, dans laquelle l'algorithme d'apprentissage automatique est entraîné à l'aide d'une perte de probabilité.

14. Méthode selon la revendication 13, dans laquelle la perte de probabilité est une fonction de perte de probabilité dérivée gaussienne.

15. Méthode selon la revendication 9, dans laquelle l'algorithme de réduction de bruit est un algorithme de réduction de bruit basé sur un réseau neuronal convolutif.
